# EUROPEAN PATENT APPLICATION

(11) **EP 4 624 452 A1**
(43) Date of publication of application: **01.10.2025**
(21) Application number: 23905864.7
(22) Date of filing: 18.12.2023
(51) Int. Cl.: C07C 291/04, A61K 9/127, A61K 47/18, A61K 47/28, A61K 47/22, A61K 47/24

(54) **AMPHIPATHIC LIPID CONTAINING TERTIARY AMINE N-OXIDE GROUP, LIPOSOME DRUG DELIVERY SYSTEM AND USE**

(30) Priority: 22.12.2022 CN 202211658103
(71) Applicant: Hangzhou Tito Biotechnology Partnership Enterprise (Limited Partnership), Hangzhou, Zhejiang Province 311222 (CN)
(72) Inventor: SHEN, Youqing, Hangzhou, Zhejiang 310058 (CN)
(74) Representative: Elzaburu S.L.P.
(86) International application number: PCT/CN2023/139379
(87) International publication number: WO 2024/131700

(57) **Abstract**

An amphiphilic lipid including a tertiary amine N-oxide group, a liposomal drug-delivery system, and a use of the amphiphilic lipid are provided. The amphiphilic lipid is a compound shown in a formula I, where R and R' each are independently selected from C₁-C₄ alkyl and X is a hydrophobic unit. The amphiphilic lipid can be used alone or together with the traditional phospholipid to prepare a liposomal drug-delivery system. The liposomal drug-delivery system can significantly prolong the blood circulation time of a drug and increase the accumulation and penetration of a drug in target tissues, resulting in a significantly-improved therapeutic effect.

## Description

### TECHNICAL FIELD

The present disclosure relates to the technical field of medicine, and in particular to an amphiphilic lipid including a tertiary amine N-oxide group, a liposomal drug-delivery system, and a use of the amphiphilic lipid.

### BACKGROUND

Liposomes are phospholipid bilayer vesicles produced by encapsulating a hydrophilic nucleus with one or more concentric phospholipid bilayers (Riley et al., Nature Reviews Drug Discovery, 2019, 18, 175-196). Liposomes have diversified structures, excellent biocompatibility, no toxicity, and no immunogenicity. As a result, liposomes are excellent drug delivery vehicles (Shah et al., Advanced Drug Delivery Reviews, 2020, 156, 4-22). Liposomal nanodrugs have been extensively used in the treatment of a variety of diseases, such as Doxil^{®} (liposomal doxorubicin) and Onivyde^{®} (liposomal irinotecan). Liposomal formulations can prevent the leakage and rapid clearance of drugs. However, growing evidence reveals that liposomes prepared from the traditional phospholipids have disadvantages such as insufficient accumulation in target tissues and poor permeability, resulting in limited therapeutic efficacy (Zhou et al., Biomaterials, 2020, 240, 119902). Designing and optimizing liposomal formulations to overcome the complicated physiological or pathological barriers in the human body, especially those restricting the accumulation or permeation in target tissues during drug delivery, remains a huge challenge.

Based on the transcytosis mechanism of cells themselves, active transcellular transport-type nanodrugs enable cells to expel a portion of a drug while continuously engulfing the drug, such that the drug can be transported across blood vessels to reach each target cell, which significantly improves the accumulation and penetration of the drug in target tissues (Zhou et al., Nature Nanotechnology, 2019, 14, 799). The design of active transcellular transport-type liposomal drug-delivery systems to improve the accumulation and penetration of the traditional liposomal formulations in target tissues is of great significance for improving the efficacy of liposomal drug-delivery systems.

Recent studies have shown that tertiary amine N-oxide group-derived nanodrugs can effectively induce the active transcellular transport between tumor endothelial cells and tumor cells. This is because these materials demonstrate strong affinity for phospholipids on cell membranes, are easily adsorbed on cell surfaces and thus endocytosed, and can target the Golgi apparatus of cells and thus be packaged and exported by cells (Chen et al., Nature Biomedical Engineering, 2021, 5, 1019). Moreover, tertiary amine N-oxide groups possess simple and diversified structures. Through structural modifications, a variety of tertiary amine N-oxide group-containing lipids can be produced to meet the therapeutic needs of different diseases. Therefore, the development of tertiary amine N-oxide group-containing lipids and the use of these tertiary amine N-oxide group-containing lipids in liposomal drug-delivery systems have great potential for clinical transformation.

### CONTENT OF THE INVENTION

An objective of the present disclosure is to provide an amphiphilic lipid composed of a tertiary amine N-oxide group, a liposomal drug-delivery system, and a use of the amphiphilic lipid. The amphiphilic lipid can replace all or part of the phospholipid components in the traditional liposomal formulations. The liposomal drug-delivery system based on the amphiphilic lipid can significantly prolong the blood circulation time of a drug and increase the accumulation and penetration of a drug in target tissues, resulting in a significantly-improved therapeutic effect.

To solve the technical problems, the present disclosure adopts the following technical solutions:
An amphiphilic lipid including a tertiary amine N-oxide group is provided, where the amphiphilic lipid is a compound shown in a formula I: 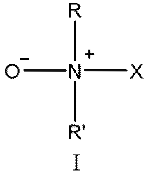
where R and R' each are independently selected from C₁-C₄ alkyl and X is a hydrophobic unit.

Preferably, when the hydrophobic unit X is a hydrophobic aliphatic chain, the amphiphilic lipid is a compound shown in a formula II or a formula III: 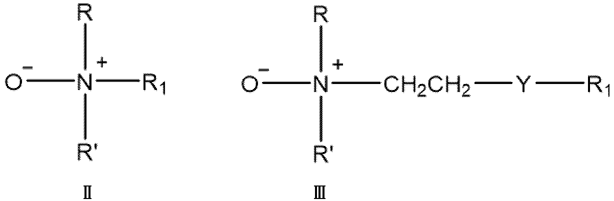 where R₁ is one or more of C₅₋₃₂ alkyl, cholesterol, and cholic acid; and Y is one or more of an ester group, a carbonate group, amido, a carbamate group, ureido, an ether group, sulfonyl, sulfinyl, and aryl. C₅₋₃₂ alkyl is amyl, octyl, decyl, dodecyl, octadecyl, etc., for example.

Preferably, an amphiphilic lipid including the hydrophobic aliphatic chain includes the following compounds:

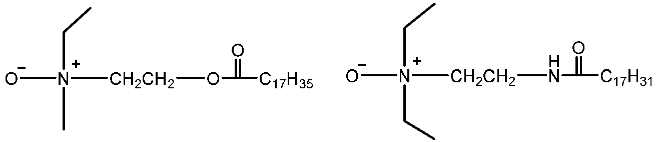

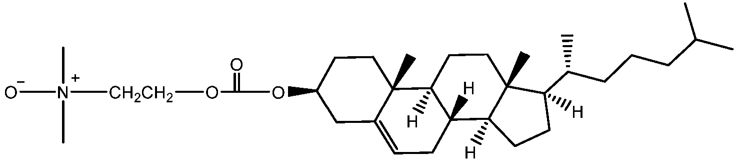

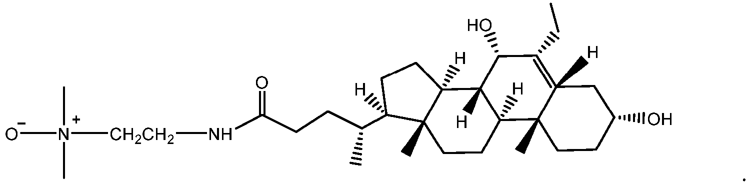

Preferably, the hydrophobic unit X includes two hydrophobic chains.

Further, an amphiphilic lipid including the two hydrophobic chains is a compound shown in a formula IV, a formula V, a formula VI, or a formula VII: 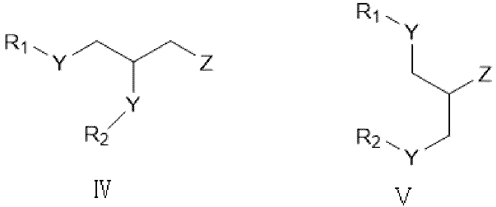 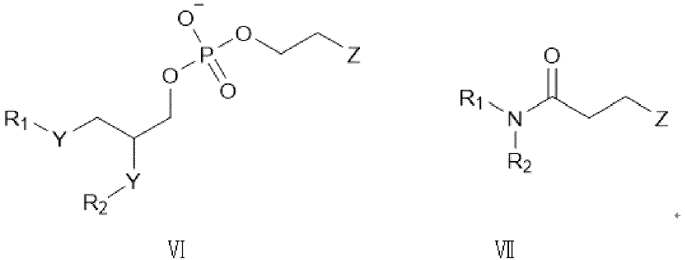 where R₁ is one or more of C₅₋₃₂ alkyl, cholesterol, and cholic acid; R₂ is one or more of C₅₋₃₂ alkyl, cholesterol, and cholic acid; Y is one or more of an ester group, a carbonate group, amido, a carbamate group, ureido, an ether group, sulfonyl, sulfinyl, and aryl; and Z is a tertiary amine N-oxide structural unit.

Preferably, the tertiary amine N-oxide structural unit is selected from one of the following: 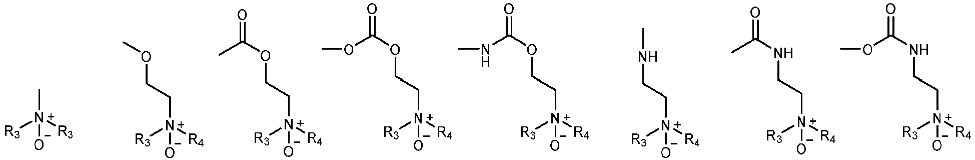 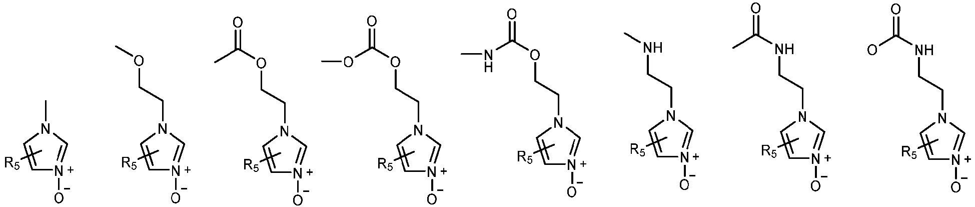 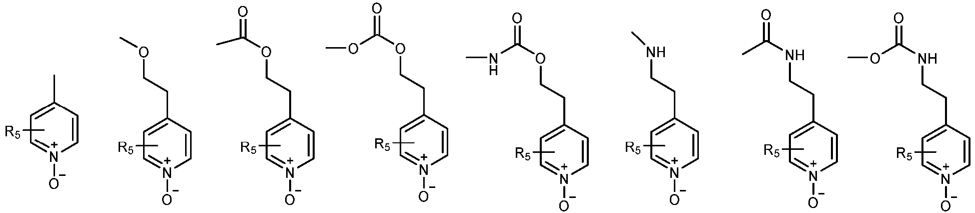 where R₃ and R₄ each are independently selected from one or more of C₁-C₄ alkyl, substituted alkyl, aryl, and substituted aryl; R₅ is selected from one or more of C₁-C₄ alkyl, substituted alkyl, aryl, substituted aryl, and a heteroatomic group; and the heteroatomic group includes a halogen, hydroxyl, and cyano.

In the present disclosure, the substituted alkyl includes hydroxyalkyl, haloalkyl, nitroalkyl, cyanoalkyl, alkylbenzene, etc, the aryl includes phenyl, naphthyl, anthryl, fluorenyl, pyridyl, benzothienyl, benzofuryl, indolyl, quinolyl, etc., and a substituent of the substituted aryl includes hydroxyl, a halogen, nitro, cyano, naphthyl, etc.

Preferably, the substituent on the tertiary amine N-oxide structural unit is dimethyl or diethyl, and a liposome prepared accordingly has a small particle size.

Further preferably, the amphiphilic lipid is an N-oxide-N,N-dimethyl or N-oxide-N,N-diethyl-based amphiphilic lipid synthesized with 2,2-bis(hydroxymethyl)propionic acid (BHP) as a linker unit. The synthesis of the amphiphilic lipid is controllable and simple, and the amphiphilic lipid can be degraded. The amphiphilic lipid has a structural formula as follows: 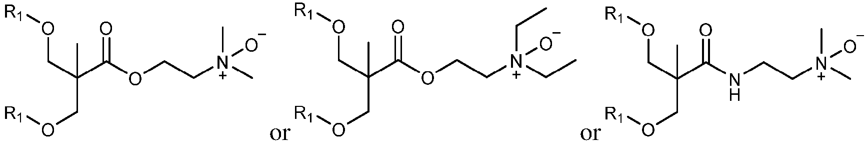 where R₁ is C₅₋₃₂ alkyl (a fatty acid chain).

Further preferably, R₁ is C₁₈ alkyl, and the amphiphilic lipid has a structural formula as follows: 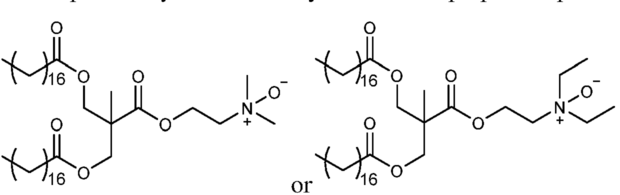 The amphiphilic lipid can readily produce a stable liposome either by itself or in combination with other lipids.

A liposome prepared from an amphiphilic lipid including a tertiary amine N-oxide group is provided, where the liposome is prepared from one or more amphiphilic lipids including the tertiary amine N-oxide group; or
the liposome is prepared from one or more amphiphilic lipids including the tertiary amine N-oxide group and a lipid without the tertiary amine N-oxide group.

A mass ratio of the amphiphilic lipids including the tertiary amine N-oxide group to the lipid without the tertiary amine N-oxide group is 1:(0-10).

The amphiphilic lipid including the tertiary amine N-oxide group can replace the phospholipid components in the traditional liposomal formulations. The liposome based on the amphiphilic lipid can significantly prolong the blood circulation time of a drug and increase the accumulation and intratumoral penetration of a drug in target tissues.

Further preferably, the lipid without the tertiary amine N-oxide group is selected from one or more of cholesterol, a phospholipid, D-α-tocopheryl polyethylene glycol succinate, and a cationic lipid. The cationic lipid includes one or more of 1,2-dioleoyl-3-trimethylammonium propane (DOTAP), trimethyl[2,3-(dioleyloxy)propyl]ammonium chloride (DOTMA), and 3β-[N-(N',N'-dimethylaminoethane)-carbamoyl]cholesterol (DC-Chol).

A liposomal drug-delivery system including the liposome and a drug is provided, where the liposome serves as a carrier to encapsulate the drug.

Preferably, a mass ratio of the drug to the liposome is (0.01-0.5):1, and the drug includes, but is not limited to, an anti-tumor drug.

The anti-tumor drug is one or more of doxorubicin, epirubicin, camptothecin, 7-ethyl-10-hydroxycamptothecin, irinotecan, paclitaxel, oxaliplatin, gemcitabine, and curcumin.

A use of the amphiphilic lipid including a tertiary amine N-oxide group in preparation of a liposomal drug-delivery system is provided.

The present disclosure provides a method for preparing a liposome from an amphiphilic lipid including a tertiary amine N-oxide group, including the following steps:
step 1: preparation of a liposomal membrane: dissolving the amphiphilic lipid, and cholesterol and/or D-α-tocopheryl polyethylene glycol succinate in a solvent, and concentrating at 30°C to 45°C to form the membrane; and
step 2: hydration: adding phosphate-buffered saline (PBS) to the liposomal membrane, and hydrating at 4°C to 50°C for 12 h to 48 h.
Drug loading: For a hydrophobic drug, in the step 1, the drug is dissolved together with the lipid in the solvent to prepare the liposomal membrane. For a hydrophilic drug, in the step 2, the drug is dissolved in the PBS and added to the prepared liposomal membrane.

For the components, a mass ratio of the cholesterol to the amphiphilic lipid is (0-1):1, and a mass ratio of the D-α-tocopheryl polyethylene glycol succinate to the amphiphilic lipid is (0-1):1.

The solvent is one or more of dichloromethane, trichloromethane, tetrahydrofuran (THF), methanol, and ethanol.

The present disclosure has the following beneficial effects:
The present disclosure applies amphiphilic lipids including tertiary amine N-oxide groups to the field of drug delivery, and can provide a class of excellent candidate materials for this field. The existing liposomes in the art can only play the role of prolonging the blood circulation time. In contrast, while significantly extending the blood circulation time, the superhydrophilicity of the tertiary amine N-oxide group in the amphiphilic lipid including the tertiary amine N-oxide group of the present disclosure can interact with phospholipids of vascular endothelial cells and target cells of target tissues to effectively induce the transcellular transport, such that a drug can be effectively exuded from lesion blood vessels and enter the target tissues, and can be actively transported in the tissues through transcytosis among target cells, which significantly enhances the efficacy.

Further, the tertiary amine N-oxide group-containing liposomes enter cells in different ways from the traditional liposomes in the art, and are highly selectively enriched in the endoplasmic reticulum or Golgi apparatus of cells after entering cells, which can avoid the destruction of drugs by lysosomes and improve the utilization of drugs by cells.

A method for encapsulating a drug with the tertiary amine N-oxide group-containing liposome of the present disclosure is simple and efficient, and the common method in the art can be adopted.

The amphiphilic lipid including a tertiary amine N-oxide group has a clear structure, and a synthesis method of the amphiphilic lipid is simple and merely involves the three simple chemical reactions of acylation, esterification, and oxidation. By adjusting the structures of the hydrophobic aliphatic chain and the hydrophilic tertiary amine N-oxide, lipid molecules suitable for treating various physiological diseases can be obtained.

### BRIEF DESCRIPTION OF THE DRAWINGS

FIG. 1 shows a synthetic reaction for 18-BHP-ODMA in Example 1;
FIG. 2 shows proton nuclear magnetic resonance spectroscopy characterization results of 18-BHP-ODMA and 18-BHP-ODEA in Example 1;
FIG. 3 shows a matrix-assisted laser desorption/ionization time-of-flight mass spectrometry (MALDI-TOF-MS) spectrum of 18-BHP-ODMA in Example 1;
FIG. 4 shows an MALDI-TOF-MS spectrum of 18-BHP-ODEA in Example 1;
FIG. 5 shows particle size distributions of liposomes in Example 2;
FIG. 6 shows particle sizes of liposomes after being incubated in different media for 24 h in Test Example 1;
FIG. 7 shows particle size changes of liposomes after being stored in a 4°C freezer for different time periods in Test Example 1;
FIG. 8 shows results of cytotoxicity tests of liposomal formulations in Test Example 2;
FIG. 9 shows the comparison of ^{DiI}ODML and ^{DiI}ODEL in a HepG2 cell line with an endoplasmic reticulum dye in Test Example 3;
FIG. 10 shows the comparison of ^{DiI}ODML and ^{DiI}ODEL in a HepG2 cell line with a Golgi apparatus dye in Test Example 3;
FIG. 11 shows results of transcellular transport tests of ^{DiI}ODML and ^{DiI}ODEL in Test Example 4;
FIG. 12 shows *in vivo* imaging results of blood of mice at 48 h after ^{DiR}ODML or ^{DiR}ODEL is injected in Test Example 5;
FIG. 13 shows *in vivo* imaging results of major tissues of mice at 48 h after ^{DiR}ODML or ^{DiR}ODEL is injected in Test Example 5;
FIG. 14 shows tumor inhibition curves of liposomal formulations for a HepG2 tumor-bearing mouse model in Test Example 6; and
FIG. 15 shows body weight change curves of HepG2 tumor-bearing mice in Test Example 6.

### DETAILED DESCRIPTION OF THE EMBODIMENTS

The technical solutions of the present disclosure will be described in further detail below with reference to specific embodiments.

In the present disclosure, unless otherwise specified, all raw materials and devices adopted are commercially available or are commonly used in the art. All methods in the following embodiments are the conventional methods in the art, unless otherwise specified. The compounds in the present disclosure can be prepared by a variety of synthetic methods well known to those skilled in the art, including the specific embodiments listed below, the embodiments produced by combining these embodiments with other compound synthesis methods, and equivalent alternatives well known to those skilled in the art, and are also commercially available. Preferred embodiments include, but are not limited to, the embodiments of the present disclosure. It will be apparent to those skilled in the art that various changes and modifications can be made to the specific embodiments of the present disclosure without departing from the spirit and scope of the present disclosure.

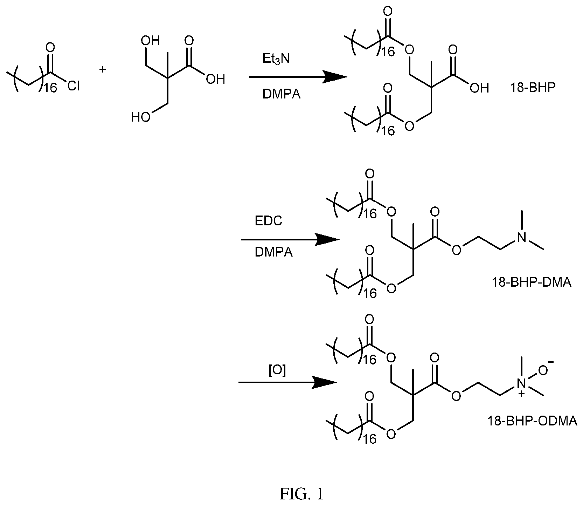

### Example 1 Synthesis of 18-BHP-ODMA and 18-BHP-ODEA (FIG. 1)

### 1.1 Synthesis of 18-BHP

BHP (10.05 g, 75 mmol), 4-dimethylaminopyridine (DMAP, 0.3 g, 2.5 mmol), and triethylamine (23.6 mL, 170 mmol) were added to a round-bottomed flask with 200 mL of anhydrous THF. In an ice bath, stearyl chloride (49.98 g, 165 mmol) was slowly added dropwise. After the dropwise addition was completed, warming was conducted to room temperature, and stirring was conducted for 24 h. After the reaction was completed, the THF in the system was removed through rotary evaporation. A residue was dissolved with 250 mL of dichloromethane, washed with 1 M dilute hydrochloric acid (80 mL × 2), deionized water (80 mL × 3), and a saturated sodium chloride solution (80 mL × 3), and dried with anhydrous magnesium sulfate. All solvents were removed through rotary evaporation. Recrystallization was conducted with acetone as a solvent to produce a white solid. The white solid was placed overnight in a vacuum chamber. The white solid was the product 18-BHP (49.64 g, yield: 89.7%).

### 1.2 Synthesis of 18-BHP-DMA

18-BHP (3 g, 4 mmol), DMAP (49 mg, 0.4 mmol), and 1-ethyl-3-(3-dimethylaminopropyl)carbodiimide hydrochloride (EDC, 1.53 g, 8 mmol) were added to a round-bottomed flask, 50 mL of dichloromethane was added, and stirring was conducted for 15 min. N,N-dimethylethanolamine (357 mg, 4 mmol) was added, and stirring was conducted overnight. After a reaction was completed, a reaction solution was washed with deionized water (50 mL × 2) and a saturated sodium chloride solution (50 mL × 2), dried with anhydrous magnesium sulfate, concentrated through rotary evaporation, and subjected to separation through silica gel-based column chromatography with an n-hexane solution including 50% of ethyl acetate as a mobile phase. An eluate was spin-dried and concentrated, and placed overnight in a vacuum chamber to produce a white solid, which was the product 18-BHP-DMA (1.93 g, yield: 58.1%).

### 1.3 Synthesis of 18-BHP-DEA

18-BHP (3 g, 4 mmol), DMAP (49 mg, 0.4 mmol), and EDC (1.53 g, 8 mmol) were added to a round-bottomed flask, 50 mL of dichloromethane was added, and stirring was conducted for 15 min. N,N-diethylethanolamine (468 mg, 4 mmol) was added, and stirring was conducted overnight. After the reaction was completed, the reaction solution was washed with deionized water (50 mL × 2) and a saturated sodium chloride solution (50 mL × 2), dried with anhydrous magnesium sulfate, concentrated through rotary evaporation, and subjected to separation through silica gel-based column chromatography with an n-hexane solution including 30% of ethyl acetate as a mobile phase. An eluate was spin-dried and placed overnight in a vacuum chamber to produce a white solid, which was the product 18-BHP-DEA (2.11 g, yield: 61.5%).

### 1.4 Synthesis of 18-BHP-ODMA or 18-BHP-ODEA

18-BHP-DMA (1.11 g, 1.5 mmol) or 18-BHP-DEA (1.15 g, 1.5 mmol) was added to a round-bottomed flask with 5 mL of dichloromethane. Then meta-chloroperoxybenzoic acid (0.31 g, 1.8 mmol) was dissolved in 5 mL of dichloromethane and slowly added dropwise to a solution produced above in an ice bath. After the dropwise addition was completed, the ice bath was removed, and the reaction was further allowed for 3 h. After the reaction was completed, the reaction solution was subjected to separation through basic aluminum oxide-based column chromatography with a dichloromethane solution including 10% of methanol as a mobile phase. An eluate was spin-dried and placed overnight in a vacuum chamber to produce a white solid, which was the product 18-BHP-ODMA (1.04 g, yield: 92.0%) or 18-BHP-ODEA (1.12 g, 95.1%).

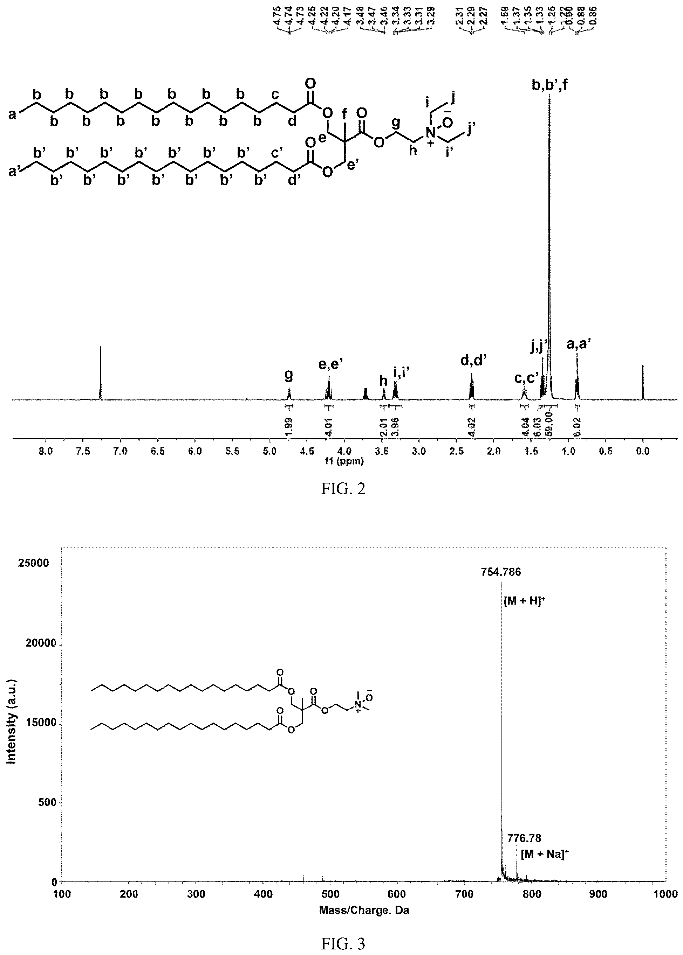

18-BHP could react directly with 2-(N-oxide-N,N-dimethyl)ethanol or 2-(N-oxide-N,N-diethyl)ethanol 2 to prepare 18-BHP-ODMA or 18-BHP-ODEA. Proton nuclear magnetic resonance spectroscopy or MALDI-TOF-MS characterization results of 18-BHP-ODMA or 18-BHP-ODEA were shown in FIG. 2 to FIG. 4.

The stearyl chloride in the step 1.1 could be replaced with an equal molar mass of palmitoyl chloride, myristoyl chloride, or lauroyl chloride to produce an intermediate X-BHP (X represents a carbon chain length) with a carbon chain length of 16, 14, or 12. Then the 18-BHP in the step 1.2 or 1.3 was replaced with an equal molar mass of X-BHP, and/or the ethanolamine in the step 1.2 or 1.3 was replaced with an equal molar mass of N,N-dibutylethanolamine (DBU) or 4-(2-hydroxyethyl)pyridine (PY). Finally, according to the step 1.4, oxidation was conducted to produce lipids X-BHP-ODMA, X-BHP-ODEA, X-BHP-ODBU, and X-BHP-OPY with different carbon chain lengths and different tertiary amine N-oxide groups.

### Example 2 Preparation of tertiary amine N-oxide group-containing liposomes (liposomes prepared from amphiphilic lipids including tertiary amine N-oxide groups)

10 mg of 18-BHP-ODMA or 18-BHP-ODEA, and 2.5 mg of cholesterol and/or 2.5 mg of D-α-tocopheryl polyethylene glycol succinate were weighed and added to a flask, 9 mL of trichloromethane and 1 mL of methanol were added, and an ultrasonic treatment was conducted for dissolution. Rotary evaporation was conducted under reduced pressure to form a dry lipid membrane on a wall of the flask. Then 5 mL of PBS was added, and hydration was allowed at room temperature for 1 h. Finally, the resulting solution was filtered through the membrane (200 nm) to produce a blank tertiary amine N-oxide group-containing liposome, which was named ODML or ODEL.

The 18-BHP-ODMA or 18-BHP-ODEA could be replaced with other tertiary amine N-oxide group-containing lipids (at an equal molar mass) in Example 1 to produce other tertiary amine N-oxide group-containing liposomes.

### Example 3 Preparation of fluorescently-labeled tertiary amine N-oxide group-containing liposomes

10 mg of 18-BHP-ODMA or 18-BHP-ODEA, 2.5 mg of cholesterol and/or 2.5 mg of D-α-tocopheryl polyethylene glycol succinate, and 100 µg of DiI or DiR were weighed and added to a flask, 9 mL of trichloromethane and 1 mL of methanol were added, and an ultrasonic treatment was conducted for dissolution. Rotary evaporation was conducted under reduced pressure to form a dry lipid membrane on the wall of the flask. Then 0.5 mL of the prepared 7-ethyl-10-hydroxycamptothecin/irinotecan nanoparticles and 4.5 mL of PBS were added, and hydration was allowed at room temperature for 1 h. The free fluorescent molecules were removed through the separation of a sephadex column. Accordingly, a DiI or DiR-labeled nitrogen oxide-containing liposome ^{DiI}ODML, ^{DiI}ODEL, ^{DiR}ODML, or ^{DiR}ODEL was produced.

Example 4 The preparation of tertiary amine N-oxide group-containing liposomes loaded with 7-ethyl-10-hydroxycamptothecin/irinotecan nanoparticles and the preparation of 7-ethyl-10-hydroxycamptothecin/irinotecan nanoparticles (SC) were conducted with reference to the literature (Hu et al., Journal of Controlled release, 2015, 220, 175-179).

10 mg of 18-BHP-ODMA or 18-BHP-ODEA, and 2.5 mg of cholesterol and/or 2.5 mg of D-α-tocopheryl polyethylene glycol succinate were weighed and added to a flask, 9 mL of trichloromethane and 1 mL of methanol were added, and an ultrasonic treatment was conducted for dissolution. Rotary evaporation was conducted under reduced pressure to form a dry lipid membrane on the wall of the flask. Then 0.5 mL of the prepared 7-ethyl-10-hydroxycamptothecin/irinotecan nanoparticles and 4.5 mL of PBS were added, and hydration was allowed at room temperature for 1 h. The unloaded 7-ethyl-10-hydroxycamptothecin/irinotecan nanoparticles were removed through the separation of a sephadex column. Accordingly, a tertiary amine N-oxide group-containing liposome loaded with 7-ethyl-10-hydroxycamptothecin/irinotecan was produced, which was named ODMLSC or ODELSC.

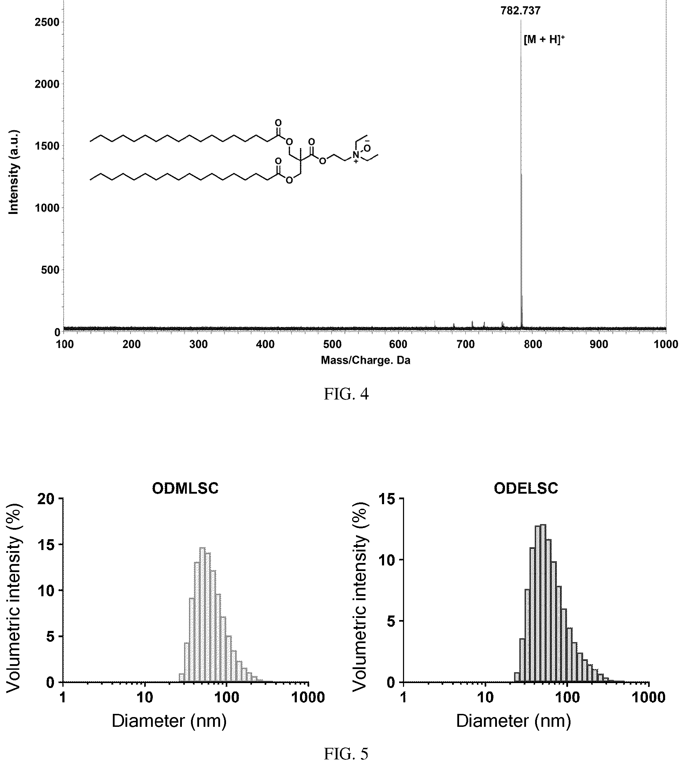

Particle size distribution patterns of ODML, ODEL, ODMLSC, and ODELSC were shown in FIG. 5.

### Test Example 1 Stability testing for liposomal formulations

1 mL of each liposome solution in Example 4 was pipetted and added to 4 mL of deionized water, PBS, HEPES, DMEM, or fetal bovine serum (FBS), and incubated under shaking at 37°C and 100 rpm for 24 h. Then the particle size of a liposome was determined by a dynamic light scattering (DLS) particle size analyzer. For long-term stability testing, each liposome was stored in a 4°C freezer. A sample was collected at a set time point and tested for a particle size by DLS.

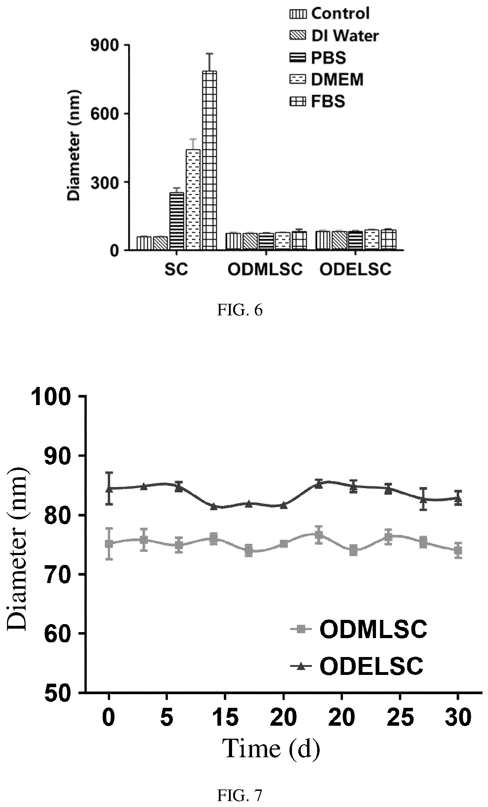

Test results were shown in FIG. 6 to FIG. 7. The nitrogen oxide-containing liposomes exhibit excellent stability in deionized water, PBS, HEPES, DMEM, or FBS, and can remain stable during long-term storage.

### Test Example 2 Cytotoxicity test

Cells were added at 5,000 cells/well to a 96-well plate. 100 µL of a medium was added to each well. A culture was conducted for 24 h in a 37°C incubator with a CO₂ concentration of 5% and a humidity of 95%. 100 µL of a drug was added at different concentrations to each well, including SC, ODMLSC, or ODELSC. A final concentration of a drug molecule in each well was an experimental design value. 100 µL of a fresh medium was added in a blank group. After a culture was conducted for 48 h, centrifugation was conducted at 1,100 rpm for 6 min, and a medium in each well was discarded. 100 µL of an MTT working solution was added, and a culture was continued for 3 h. Centrifugation was conducted at 3,300 rpm for 5 min, and the MTT working solution in each well was discarded. 100 µL of dimethylsulfoxide (DMSO) was added, and shaking was conducted for 5 min to make crystals in each well fully dissolved. An absorbance of a sample at 562 nm was detected by a microplate reader. Each set of data was an average of results of three independent experiments for a same sample.

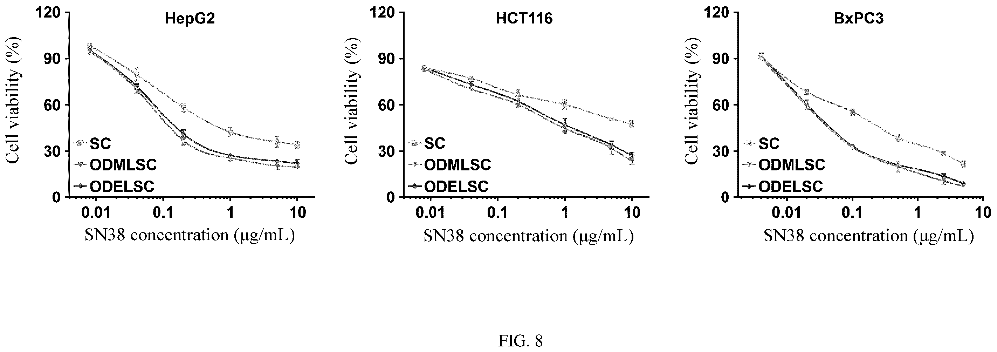

Test results were shown in FIG. 8. The tertiary amine N-oxide group-containing liposomes themselves have no significant toxicity at a cellular level and exhibit prominent biocompatibility, making these liposomes perfect drug delivery vehicles. Notably, ODMLSC or ODELSC shows much greater cytotoxicity than SC itself, and has a much smaller IC₅₀ value than SC (Table 1), indicating that the tertiary amine N-oxide group-containing liposomes can increase the toxicity of loaded drugs to cells.

**Table 1 IC₅₀ values of SC, ODMLSC, or ODELSC in different cell lines (unit: µg/mL)**

| | HepG2 | HCT116 | BxPC3 |
|---|---|---|---|
| SC | 0.98±0.27 | 5.76±0.31 | 0.40±0.02 |
| ODMLSC | 0.17±0.01 | 0.54±0.08 | 0.09±0.01 |
| ODELSC | 0.22±0.03 | 0.74±0.12 | 0.10±0.01 |

### Test Example 3 Subcellular distribution of tertiary amine N-oxide group-containing liposomes

100,000 HepG2 cells were plated in a confocal dish and incubated overnight until adherent. Then the original medium was replaced with a fresh medium. 0.2 µL of an endoplasmic reticulum or Golgi apparatus fluorescent dye was added, and incubation was conducted for 30 min. Then a nuclear fluorescent dye Hoechst was added, and incubation was further conducted for 15 min. Finally, ^{DiI}ODML or ^{DiI}ODEL was added with a final concentration of DiI in a medium being 0.5 µg/mL, and incubation was conducted for 60 min. Images were acquired by a laser scanning confocal microscope, and a colocalization rate between liposomes and cellular microsomes was calculated with imageJ.

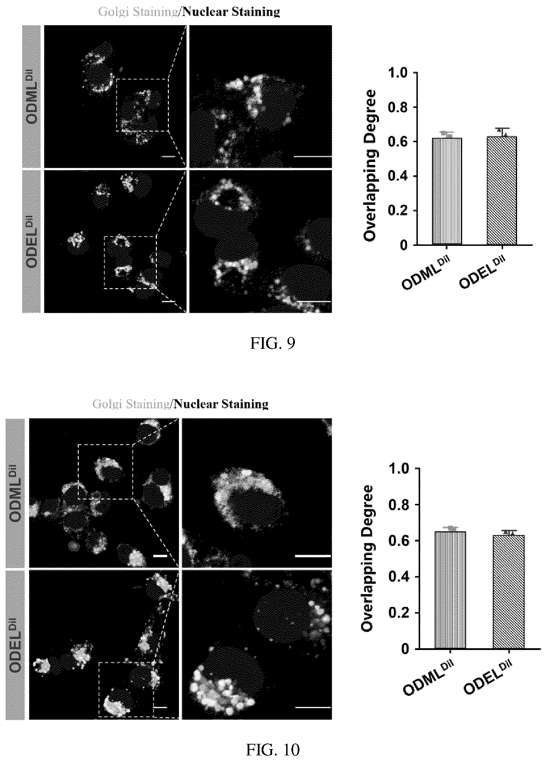

Test results were shown in FIG. 9 to FIG. 10. The distributions of ^{DiI}ODML and ^{DiI}ODEL in the endoplasmic reticulum or Golgi apparatus in HepG2 cells are almost identical, and the corresponding colocalization rates both are higher than 60%, indicating that the tertiary amine N-oxide group-containing liposomes can target the endoplasmic reticulum or Golgi apparatus of cells.

### Test Example 4 Transcellular transport of liposomal formulations

100,000 HepG2 cells were plated in a confocal dish and incubated overnight until adherent. Then the original medium was replaced with a fresh medium. ^{DiI}ODML or ^{DiI}ODEL was added with a final concentration of DiI in a medium being 0.5 µg/mL, and incubation was conducted for 6 h. Washing was conducted with PBS. Images were acquired by a laser scanning confocal microscope. 0.8 mL of a fresh medium was then added, and a culture was conducted for 12 h. A medium was collected to culture the second batch of cells for 12 h. Then washing was conducted with PBS (including 0.5 mg/mL of heparin). Images were acquired by a laser scanning confocal microscope. This process was repeated twice.

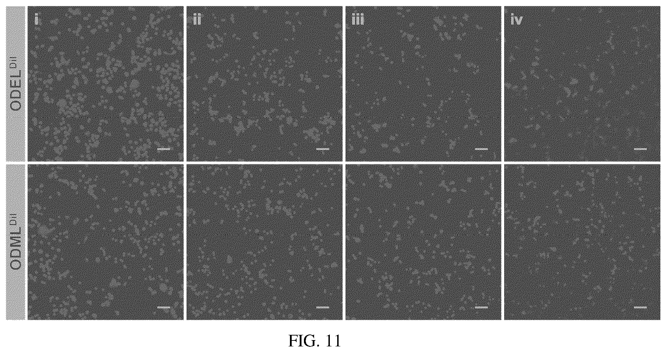

Test results were shown in FIG. 11. A strong DiI fluorescence signal can be observed in cells treated with ^{DiI}ODML or ^{DiI}ODEL in the first batch of dishes. The liposome is then partially expelled by cells and absorbed by cells in the second batch of dishes. This transcytosis continues until in the fourth batch of dishes in which a weak fluorescence signal still can be observed. It indicates that the tertiary amine N-oxide group-containing liposomes can effectively induce the transcellular transport.

### Test Example 5 Plasma clearance and tissue distribution of tertiary amine N-oxide group-containing liposomal formulations

Female BALB/c nude mice bearing HepG2 subcutaneous tumors (about 70 mm³) were randomly divided into three groups with 3 mice in each group, and intravenously injected with DiR, ^{DiR}ODML, or ^{DiR}ODEL. 24 h later, blood was collected from the orbital veins of mice. 500 µL of a blood sample was taken and tested by a small animal *in vivo* imaging instrument for a fluorescence intensity. Mice were dissected, and tissues such as a heart, a liver, a spleen, a lung, a kidney, an intestine, and a tumor were collected. Each tissue was detected by a small animal *in vivo* imaging instrument for a fluorescence intensity.

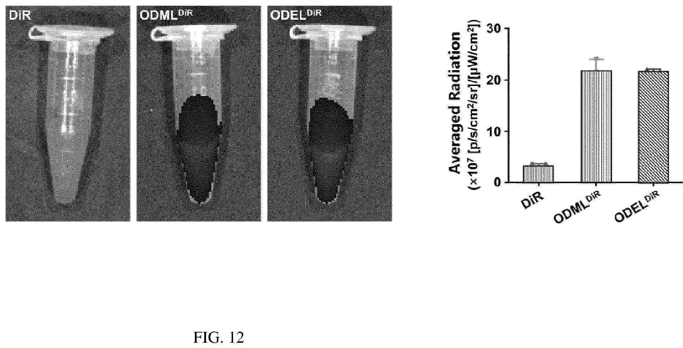

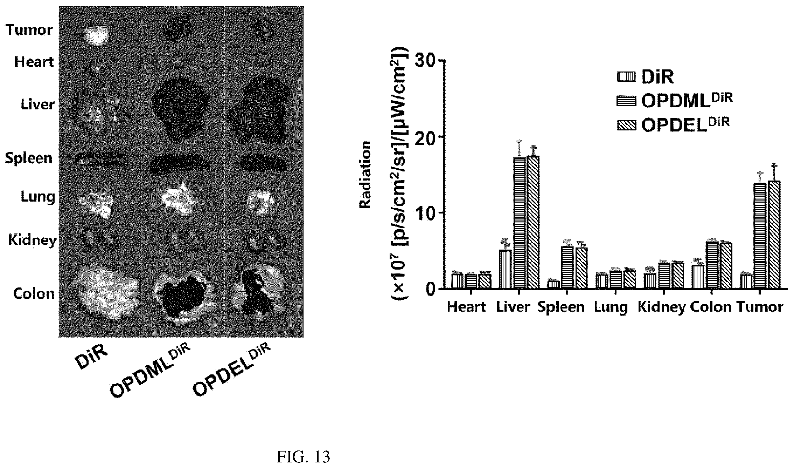

Test results were shown in FIG. 12 to FIG. 13. Almost no fluorescence signal is observed in the blood or tissues of mice in the DiR group. However, a strong DiR fluorescence signal can be observed in the blood or tissues of mice in the tertiary amine N-oxide group-containing liposome group. Quantitative statistics show that signal intensities of DiR in the blood, heart, liver, spleen, lung, kidney, intestine, and tumor of mice in the tertiary amine N-oxide group-containing liposome group all are much higher than those in the DiR group, indicating that the tertiary amine N-oxide group-containing liposome can prolong the blood circulation time of a loaded drug and increase the accumulation of a drug in a tumor tissue.

### Test Example 6 Anti-tumor activity test

Balb/c nude mice were subcutaneously injected with 2 × 10⁶ CT26 tumor cells. After a tumor grew to about 70 mm³, mice were divided into a blank control group, an SC group, an ODMLSC group, and an ODELSC group. The administration was conducted through tail vein injection every two days (Day 0, Day 2, Day 4, Day 6, and Day 8) at a dose of 10 mg/kg (SN38 equivalent). After an administration cycle was completed, mice were further observed for 19 d.

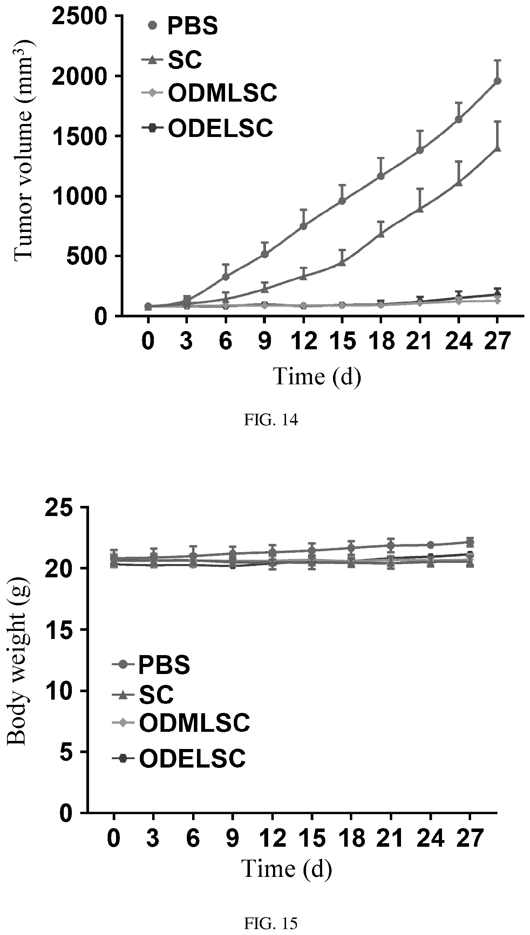

Test results were shown in FIG. 14 to FIG. 15. FIG. 14 shows that ODMLSC or ODELSC exhibits a significantly-better anti-tumor effect than SC itself. Volumes of tumors in mice of the ODMLSC or ODELSC group increase slowly during the observation period, while tumors of mice in the SC group enter an exponential growth phase immediately after the administration is stopped. Mouse tumors collected on the day when the test was completed were weighed. Results show that a tumor inhibition rate of the tertiary amine N-oxide group-containing liposome of SN38/CPT-11 is as high as 90%, which is much higher than a tumor inhibition rate (10%) of the SN38/CPT-11 nanoparticle itself. FIG. 15 shows that there is no body weight reduction in mice of the tertiary amine N-oxide group-containing liposome group of SN38/CPT-11, indicating the high biosafety of the drug.

The tertiary amine N-oxide group-containing liposome has a clear structure, and can be synthesized through simple and efficient steps. A liposomal formulation based on the tertiary amine N-oxide group-containing liposome can significantly increase a therapeutic effect of a drug, is at a leading level in the art, and has a promising application prospect.

The above examples are merely preferred solutions of the present disclosure and are not intended to limit the present disclosure in any form, and other variations and modifications may be made without departing from the technical solutions as set forth in the appended claims.

## Claims

1. An amphiphilic lipid comprising a tertiary amine N-oxide group, wherein the amphiphilic lipid is a compound shown in a formula I: 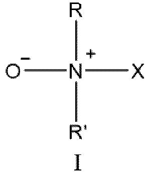 wherein R and R' each are independently selected from C₁-C₄ alkyl and X is a hydrophobic unit.

2. The amphiphilic lipid comprising the tertiary amine N-oxide group according to claim 1, wherein when the hydrophobic unit X is a hydrophobic aliphatic chain, the amphiphilic lipid is a compound shown in a formula II or a formula III: 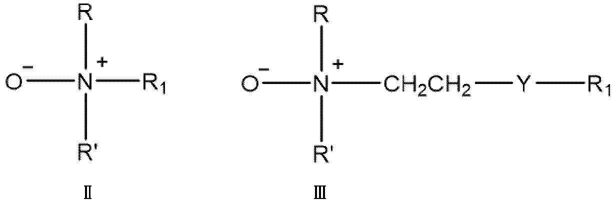 wherein R₁ is one or more of C₅₋₃₂ alkyl, cholesterol, and cholic acid; and Y is one or more of an ester group, a carbonate group, amido, a carbamate group, ureido, an ether group, sulfonyl, sulfinyl, and aryl.

3. The amphiphilic lipid comprising the tertiary amine N-oxide group according to claim 2, wherein an amphiphilic lipid comprising the hydrophobic aliphatic chain comprises the following compounds:

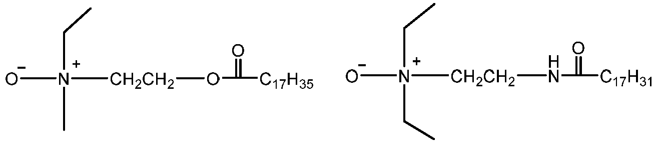

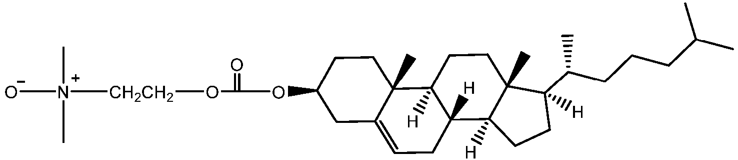

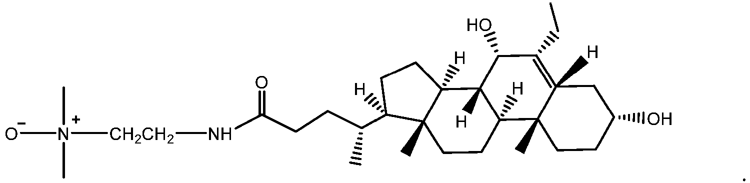

4. The amphiphilic lipid comprising the tertiary amine N-oxide group according to claim 1, wherein the hydrophobic unit X comprises two hydrophobic chains.

5. The amphiphilic lipid comprising the tertiary amine N-oxide group according to claim 4, wherein an amphiphilic lipid comprising the two hydrophobic chains is a compound shown in a formula IV, a formula V, a formula VI, or a formula VII: 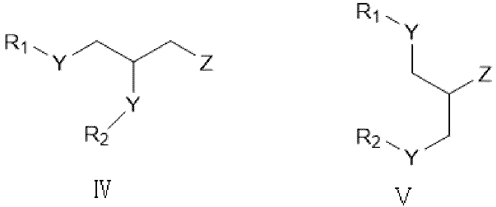 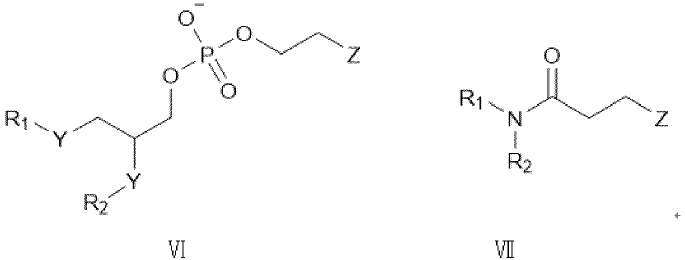 wherein R₁ is one or more of C₅₋₃₂ alkyl, cholesterol, and cholic acid; R₂ is one or more of C₅₋₃₂ alkyl, cholesterol, and cholic acid; Y is one or more of an ester group, a carbonate group, amido, a carbamate group, ureido, an ether group, sulfonyl, sulfinyl, and aryl; and Z is a tertiary amine N-oxide structural unit.

6. The amphiphilic lipid comprising the tertiary amine N-oxide group according to claim 5, wherein the tertiary amine N-oxide structural unit is selected from one of the following: 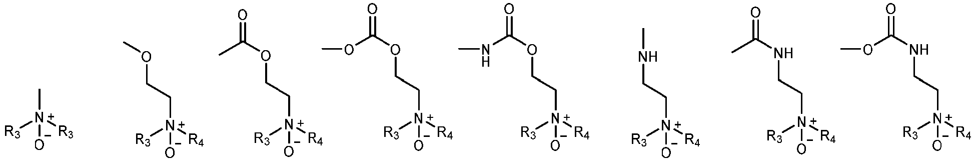 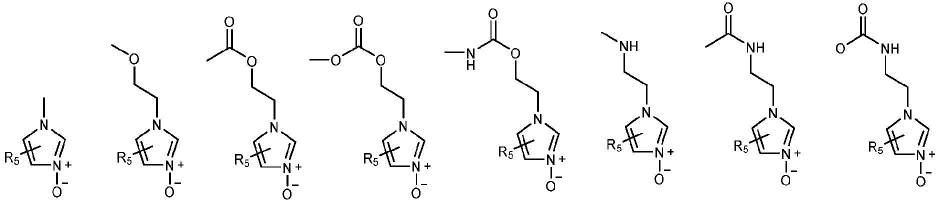 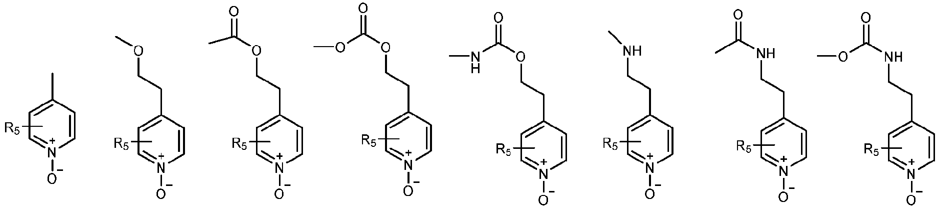 wherein R₃ and R₄ each are independently selected from one or more of C₁-C₄ alkyl, substituted alkyl, aryl, and substituted aryl; R₅ is selected from one or more of C₁-C₄ alkyl, substituted alkyl, aryl, substituted aryl, and a heteroatomic group; and the heteroatomic group comprises a halogen, hydroxyl, and cyano.

7. The amphiphilic lipid comprising the tertiary amine N-oxide group according to claim 6, wherein a substituent on the tertiary amine N-oxide structural unit is dimethyl or diethyl.

8. The amphiphilic lipid comprising the tertiary amine N-oxide group according to claim 4, wherein the amphiphilic lipid is an N-oxide-N,N-dimethyl or N-oxide-N,N-diethyl-based amphiphilic lipid synthesized with 2,2-bis(hydroxymethyl)propionic acid (BHP) as a linker unit, and has a structural formula as follows: 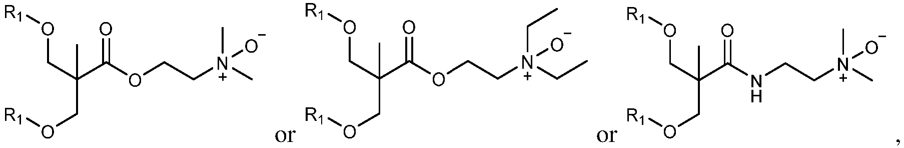 wherein R₁ is C₅₋₃₂ alkyl.

9. The amphiphilic lipid comprising the tertiary amine N-oxide group according to claim 8, wherein R₁ is C₁₈ alkyl, and the amphiphilic lipid has a structural formula as follows:

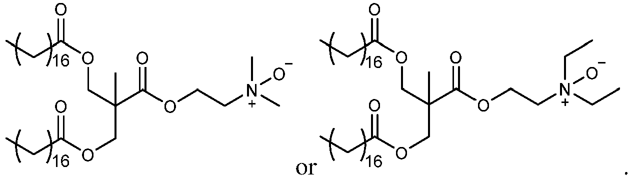

10. A liposome prepared from an amphiphilic lipid comprising a tertiary amine N-oxide group, wherein the liposome is prepared from one or more amphiphilic lipids comprising the tertiary amine N-oxide group; or
the liposome is prepared from one or more amphiphilic lipids comprising the tertiary amine N-oxide group and a lipid without the tertiary amine N-oxide group.

11. The liposome according to claim 10, wherein the lipid without the tertiary amine N-oxide group is selected from one or more of cholesterol, a phospholipid, D-α-tocopheryl polyethylene glycol succinate, and a cationic lipid.

12. A liposomal drug-delivery system comprising the liposome according to claim 10 and a drug, wherein the liposome serves as a carrier to encapsulate the drug.

13. The liposomal drug-delivery system according to claim 12, wherein a mass ratio of the drug to the liposome is (0.01-0.5): 1, and the drug comprises, but is not limited to , an anti-tumor drug.

14. The liposomal drug-delivery system according to claim 13, wherein the anti-tumor drug is one or more of doxorubicin, epirubicin, camptothecin, 7-ethyl-10-hydroxycamptothecin, irinotecan, paclitaxel, oxaliplatin, gemcitabine, and curcumin.

15. A using of the amphiphilic lipid comprising the tertiary amine N-oxide group according to any one of claim 1 to 9 in preparation of a liposomal drug-delivery system..
